# EUROPEAN PATENT SPECIFICATION

(11) **EP 2 143 703 B1**
(45) Date of publication and mention of the grant of the patent: **18.05.2011**
(21) Application number: 08012322.7
(22) Date of filing: 08.07.2008
(51) Int. Cl.: C07C 39/42, A61K 8/97, A61K 31/05, A61Q 19/00, C07C 39/17

(54) **Method for the preparation of (+)-totarol**
Verfahren zur Herstellung von (+)-Totarol
Procédé de préparation de (+)-totarol

(43) Date of publication of application: 13.01.2010
(73) Proprietor: Cognis IP Management GmbH, 40589 Düsseldorf (DE); Technische Universität Dresden, 01069 Dresden (DE)
(72) Inventor: Löhl, Thorsten, 53179 Bonn (DE); Markert, Thomas, 40789 Monheim (DE); Metz, Peter, 01309 Dresden (DE); Rogachev, Victor, 01169 Dresden (DE)

(56) References cited:
- EP-A- 1 925 301
- MIYAKE, TAKAHIRO ET AL: "Chemoenzymatic synthesis of (+)-totarol, (+)-podototarin, (+)-sempervirol, and (+)-jolkinolides E and D" TETRAHEDRON: ASYMMETRY , VOLUME DATE 2007, 18(24), 2915-2922 CODEN: TASYE3; ISSN: 0957-4166, 21 December 2007 (2007-12-21), XP002505313

## Description

The present application pertains to a method for the preparation of the tricyclic aromatic diterpene (+) - Totarol, and the use of this compound in formulations for oral applications.

(+)-Totarol, also named totara-8,11,13-trien-13-ol or 14-isopropyle-8,11,13-podo-carpatrien-13 (CAS-No. 511-15-9), molecular formula C₂₀H₃₀O, is a natural occurring diterpene which is available inter alia by extraction from the heartwood of *Podocarpus totara,* a tree indigenous in New Zealand. Totarol is also found in low amounts in other *Podocarpus, Dacrycarpus, Cupressus* and *Juniperus* species.

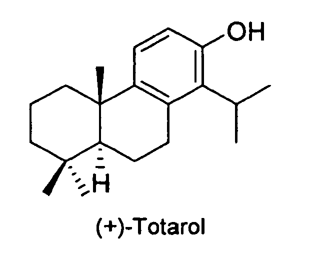

Totarol is said to have a variety of properties, like as antimicrobial agent. DE 10 2007 028506 A describes Totarol as antioxidants. U.S. 6,881,756 discloses a method for treating skin disorders relating to a method for reducing inflammation in the skin and/or treating inflammatory skin disorders, pain or pruritus by topically applying a composition comprising Totarol or pharmaceutically acceptable esters thereof. From the article of MIYAKE, TAKAHIRO ET AL: "Chemoenzymatic synthesis of (+)-totarol. (+)-podototarin, (+)-sempervirol, and (+)-iolkinolides E and D" TETRAHEDRON: ASYMMETRY, VOLUME DATE 2007, 18(24). 2915-2922 CODEN: TASYE3; ISSN: 0957-4166, 21 December 2007 a particular chemo enzymatic synthesis of (+)-

Totarol is known. The Synthesis is characterized by the starting material, which is based on a lipase-assisted resolution of a racemic primary alcohol as described on page 2916 of this article. Details as to a method for extraction and purification of (+)-Totarol are given in WO 2005/073154 A1. Besides the production of (+)-Totarol by extraction a variety of synthetic ways to prepare this compound are reported. Barltrop and Rogers developed in 1958 a first way to prepare a blend of the both enantiomers (+) and (-)-Totarol (J. Chem. Soc. 1958, 2566-2572). Marcos at al. described a synthesis, using zamoranic acid as starting compound (Tetrahedron Letters 44 (2003) 8831-8835). This approach leads to the enantiopure product (+)-Totarol, but is poor in yield.

Because of the limited natural sources for (+)-Totarol there is a constant need for other sources of this compound. It was found, that by using Sclareol or other labdan type diterpenes as starting substance, a simpler way to prepare (+)-Totarol is available.

A first embodiment of the present invention pertains to a method for the preparation of (+)-Totarol, whereby a diketon-compound according to formula (I) 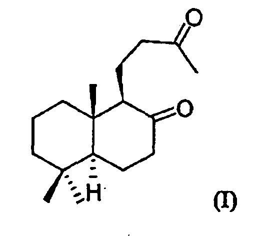 is reacted via Aldol-addition and subsequent dehydration to form the tricyclic unsaturated compound (II). Preferred is the reaction of (I) with potassium-tert-butylat, suspended in tert-butanol under an oxygen free atmosphere at the boiling temperature of the mixture; then isopropyl iodide is added and the reaction product (II) is separated. Besides the tert.-butylate other suitable bases, like sodium hydroxide or sodium methoxide, and other nonaqueous solvents may be employed; and as second step 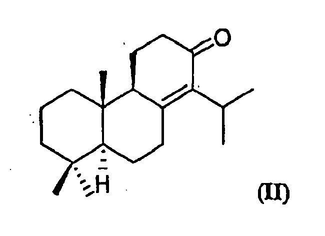 the compound (II), after separation, is suspended in acetonitrile and then reacted under a oxygen free atmosphere with a blend of LiBr and CuBr₂, heated to reflux and form (+)-Totarol (III)

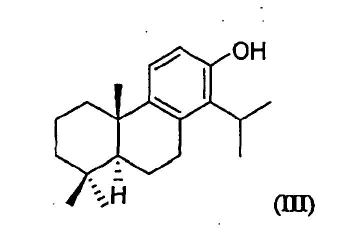

The compound (I) is an intermediate which can be derived from Labdane-type terpenes, selected from the group (-)-Sclareol, Manool or 13-epi-Manool either.

But the preferred source for (1) is (-)-Sclareol, because Manool as well as 13-epi-Manool have both a poor availability, because these substances must be isolated from plants endemic in New Zealand. Thus, the route using the better available Sclareol is advantageous.

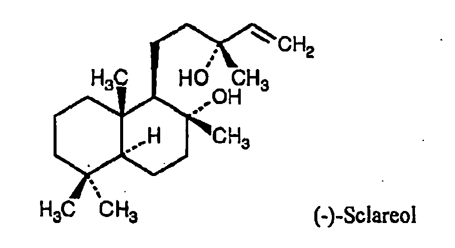

(-)-Sclareol is a natural occurring diterpene, derived from clary stage oil. *Clary sage oil* is obtained by steam distillation of flowering tops and foliage of cultivated *Salvia sclarea* L. (Lamiaceae). The quality *Broyée en vert* is obtained after cutting the fresh material and immediate distillation. Another quality named *Traditionelle* exists, which is obtained by steam distillation after drying of the tops and foliage on the field. In addition to linalyl acetate, the oil contains linalool and other terpene alcohols, as well as their acetates. When the volatile components are evaporated, a distinct ambergris note develops that is attributed to oxidative degradation products of sclareol [CAS: 515-03-7]. Sclareol is the main component in the concrete, obtained by solvent extraction of *S*. *sclarea* L. leaves.

To obtain the intermediate compound (I) the reaction sequence is as follows: As first step (-)-Sclareol is acetylated to form the corresponding diacetylsclareol (I)

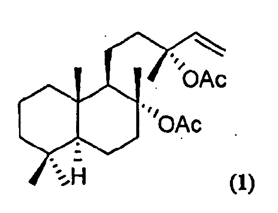

This reaction uses known methods for acetylation, and is preferably carried out in the presence of acetylchloride and dimethylanilin. As a solvent the use of dichloromethane is preferred. The reaction is preferably conducted at room temperature (21 °C). The raw product is preferably separated by extraction (for example with a blend of water/acid diethyl ether) and subsequent filtration and evaporation of solvent under low pressure conditions. Next, by a palladium catalyzed rearrangement (1) is converted into isodiacetylsclareol (2)

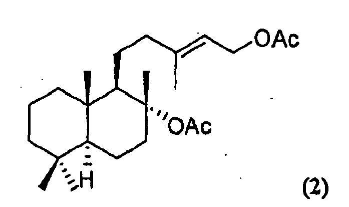

This reaction takes place under an oxygen free atmosphere, whereby Argon is the preferred gas. As Pd-catalyst PdCl₂(MeCN)₂ is the preferred one. The reaction is carried out at room temperature (21°C). The reaction needs approximately 4 to 8 h time to be complete. Subsequent to the reaction a purification step (for example by the means of column chromatography) is useful.

In general it is advantageous and therefore preferred to purify every reaction product, prior to its further use, within the described reaction sequence to obtain the maximum quality. The use of column chromatography (including HPLC) is the most preferred means for purification, together with re-crystallization.

Then the iso-diacetylsclareol (2) is subjected to cleavage preferably by ozonolysis to form the ketoacetate derivative (3)

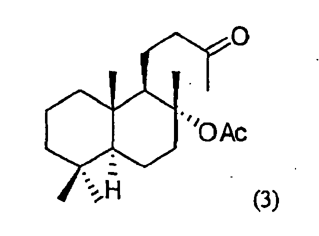

This cleavage-reaction is in a preferred embodiment carried out after suspending (2) in CH₂Cl₂, using gaseous O₃ at temperatures of about -60 °C to -80 °C. Again, purification of the raw product by means of extraction, filtration and/or chromatography is advantageous.

The ketoacetate derivate (3) is now reacted with NaHCO₃ to form the keto-olefine (4), whereby DMSO could be a useful solvent. The reaction needs elevated temperature, whereby the range from 120 °C to 170 °C is preferred. It is also preferred to conduct the reaction under an oxygen free atmosphere, like N₂ or Argon.

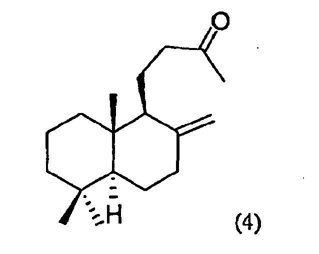

The keto-olefine (4) is at last converted into (I) by ozonolysis. The reaction conditions are similar to those used for the first ozonolysis to obtain (3). Ozonolysis is a well established method, thus the skilled reader can choose any appropriate way to carry it out on his own motion.

Reaction product (4) is converted by a one-pot cyclisation-dehydratisation reaction to form (II):

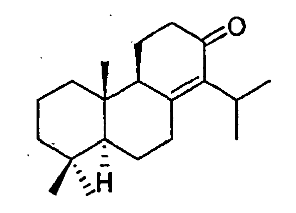

This one-pot reaction can be characterized as Aldol-addition plus subsequent dehydration, and uses in a preferred embodiment tert-butanol as solvent and potassium tert-butylate as base. The mixture of these both compounds is boiled and (4) is added. After 30 to 120 minutes iso-propyliodide solved in tert-butanol is added, and the mixture is heated under reflux for approximately 1 to 3 h. Then, purification of the raw product by means of extraction, filtration and/or chromatography is advantageous.

To convert (II) into (+)-Totarol a blend of CuBr₂ and LiBr is used, which is brought into contact with (II), solved in acetonitrile. This mixture is heated under reflux for approximately 20 to 24 h. Then the above suggested purification steps are preferred to isolate the pure compound.

As stated above it is also possible to obtain (I) from Manool or 13-epi-Manool as starting material.

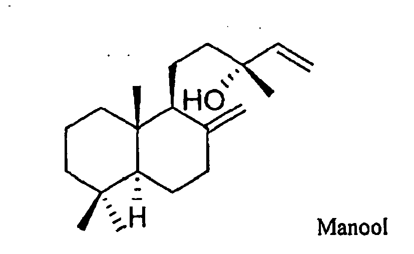

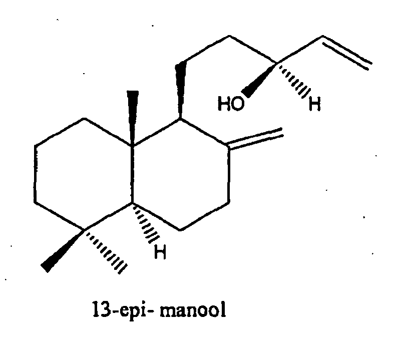

However, despite the problems concerning the availability of these substances, the process using (-)-Sclareol via the diacetate (2) is the preferred process, simply due to the fact that the overall yield of (+)-Totarol, calculated on (-)-Sclareol is greater 10 %, whereby the way using the Manool route gives only yields below 10 %.

The (+)-Totarol, prepared according to the teaching of this application is in general useful as active ingredient in cosmetic compositions, and preferably for oral care, especially in dentifrices. In addition, the (+)-Totarol, obtained by the claimed reaction sequence shows advantageous properties as anti-aging agent for skin, as biocide, and in particular as UV-protection aid, for examples in sun-screens or similar cosmetic products.

### Examples

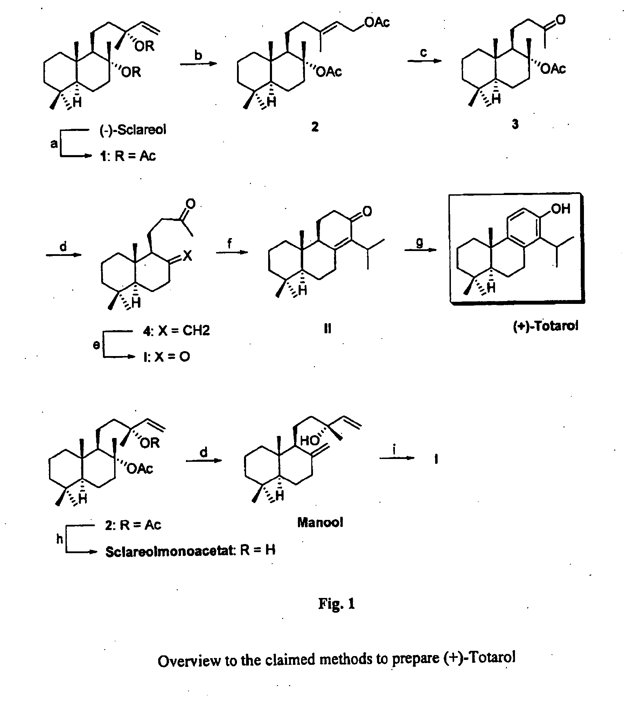

The preparation of (+)-Totarol is described in the following text. An overview about this reaction sequence is given in Fig. 1.

### Preparation of (II):

In a 500 ml three necked flask 7.605 g (67.77 mmol) potassium-*tert*-butylat has been suspended under an Argon-atmosphere in 30 ml *t*-BuOH, heated to boiling, and to this suspension a solution of 6.45 g (24.39 mmol) Diketon (I) in 70 ml *t*-BuOH at a time of 3 min has been added. The solution was boiled under reflux for 50 min. Subsequently, a solution of 3.850 g (22.65 mmol) isopropyliodide in 200 ml *t*-BuOH was added to the boiling mixture over a time of 1 h, and then the reaction mixture was boiled for another I h. After cooling to room temperature (21 °C) Et₂O (400 ml) was added, and the organic phase was washed two times with water, dried with MgSO₄ and concentrated in a vacuum. The raw-product was purified by means of column chromatography (Column: diameter 5 cm, hight 50 cm, silica gel; solvent: pentane/Acidicacidester 20:1); Yield: 3.949 g (56%); blend of 4 isomers with a molar mass of 188 in the weight ration of 9:1.5:3.5:86 (GC/MS); Appearance: orange oil.
R_{f}= 0.49 (Pentane/EtOAc 10:1).
¹H-NMR: 0.76 (s, 3H, CH₃), 0.88-1.28 (m, 4H), 0.89 (s, 3H, CH₃), 0.95 (s, 3H, CH₃), 1.15 (d, J = 7.1 Hz, 3H, CH₃), 1.21 (d, J = 7.1 Hz, 3H, CH₃), 1.42-1.73 (m, 5H), 1.78-1.97 (m, 3H), 2.06-2.40 (m, 3H), 3.10-3.23 (m, 2H).
¹³C-NMR: 14.61 (CH₃), 18.84 (CH₂), 19.67 (CH₂), 20.43 (CH₃), 21.82 (CH₃), 21.98 (CH₃), 22.45 (CH₂), 26.60 (CH₃), 31.39 (CH₂), 33.34 (C), 33.53 (CH), 37.92 (CH₂), 38.72 (CH₂), 39.93 (C), 41.80 (CH₂), 52.85 (CH), 54.43 (CH), 140.03 (C), 157.00 (C), 199.60 (CO).
GC/MS: 288 [M⁺] (75), 273 [M⁺- CH₃] (10), 245 [M⁺- C₃H₇] (10), 177 [M⁺- C₃H₇-C₄H₄O] (25), 165 (21), 152 (77), 137 (100), 123 (28).

### Preparation of (+)-Totarol (III) from (II):

In a 100 ml flask 6.420 g (28.75 mmol) CuBr₂ and 1.250 g (14.39 mmol) LiBr has been added under an Argon-atmosphere. At room temperature a solution of 3.949 g (13.689 mmol) cyclohexenone (II) in 60 ml acetonitril was added. This suspension was boiled 20 h under reflux. Then the reaction mixture was brought into contact with Et₂O (350 ml) and water (500 ml). Then the CuBr-solids are removed by sieving: The organic phase was washed five times with 200 ml 2N HCl and then with 500 ml water and dried with MgSO₄. After removal of the solvent in a vacuum the raw product was purified by means of column chromatography (column 5 x 40 cm, solvent: pentane/EtOAc 20:1) and then recrystalized in n-hexane. (+)-Totarol (III) was isolated as orange or yellow crystaline substance; Yield: 3.568 g (91%).
Melting point: 125-127°C.
R_{f}= 0.22 (pentane/EtOAc 20:1); 0.36 (pentane/EtOAc 10:1).
[α]²⁰D = +41.5 (c = 1.025, CH₂Cl₂).
¹H-NMR (500 MHz): 0.90 (s, 3H, CH₃), 0.94 (s, 3H, CH₃), 1.15-1.30 (m, 3H), 1.16 (s, 3H, CH₃), 1.33 (zwei d wie t, J = 6.7 Hz, 6H, 2CH₃), 1.43-1.47 (m wie br d, J = 13.1 Hz, 1H), 1.55-1.74 (m, 3H), 1.90 (dd, J = 7.9, 13.2 Hz, 1H), 2.21 (br d, J = 12.3 Hz, 1H), 2.70-2.77 (m, 1H), 2.93 (dd, J = 6.4, 17.1 Hz, 1H), 3.28 (m_{c},1H), 4.40 (s, 1H), 6.50 (d, J =8.5 Hz, 1H), 6.98 (d, J =8.5 Hz, 1H).
¹³C-NMR (126 MHz): 19.32 (CH₂), 19.46 (CH₂), 20.31 (CH₃), 21.56 (CH₃), 25.16 (CH₃), 27.12 (CH), 28.75 (CH₂), 33.22 (C), 33.24 (CH₃), 37.67 (C), 39.56 (CH₂), 41.53 (CH₂), 49.53 (CH), 114.24 (CH), 122.99 (CH), 130.95 (C), 133.99 (C), 143.18 (C), 151.92 (C).
GC/MS: 286 [M⁺] (40), 271 [M⁺- CH₃] (100), 243 [M⁺- C₃H₇] (10), 229 [M⁺- CH₂-C₃H₇] (10), 215 [M⁺- 2CH₂ - C₃H₇] (10), 201 [M⁺- 3CH₂ - C₃H₇] (48), 189 (27), 175 (72), 159 (12), 145 (10).

### Preparation of diacetylsclareol (1):

50.0 g (0.162 mol) Sclareol were stirred together with 98.0 g (0.809 mol) dimethyl anilin and 60 ml dichlormethane in a 500 ml flask for 30 min. The 42 g (0.531 mol) acetylchloride, dissolved in 30 ml dichlormethane where added dropwise over a period of 1 h. After addition of AcCl the mixture has been stirred for 26 h at room temperature. Subsequently, diethylether (500 ml), ice (250 g) and 2N HCl (300 ml) was added. The phases were seperated, and the organic phase was washed first with 400 ml 2N HCl and then with 100 ml saturated NaCl-solution, a saturated Na₂CO₃-solution and then again with a saturated NaCl-solution; dried with MgSO₄ and concentrated in a vacuum. The raw material was filtered with silica gel/acetoacetate. (1) was isolated in amounts of 61.310 g (156.17 mmol, 96%) as pale yellow solid substance.
R_{f}= 0.55 (pentane/EtOAc 5:1)
¹H-NMR: 0.71 (s, 3H, CH₃), 0.75 (s, 3H, CH₃), 0.79 (s, 3H, CH₃), 0.90 (dd, J = 2.1, 12.3 Hz, 2H), 1.00-1.40 (m, 7H), 1.38 (s, 3H, CH₃), 1.47 (s, 3H, CH₃), 1.50-1.70 (m, 4H), 1.80-1.88 (m, 2H), 1.87 (s, 3H, CH₃), 1.94 (s, 3H, CH₃), 2.50-2.60 (m, 1H), 5.04 (dd, J = 0.9, 6.4 Hz, 1H), 5.09 (dd, J = 0.9, 13.0 Hz, 1H), 5.89 (dd, J = 11.0, 17.5 Hz, 1H).
¹³C-NMR: 15.73 (CH₃), 18.31 (CH₂), 19.46 (CH₂), 19.94 (CH₂), 20.42 (CH₃), 21.38 (CH₃), 22.19 (CH₃), 22.87 (CH₃), 23.54 (CH₃), 33.08 (CH₃), 33.29 (C), 38.75 (CH₂), 39.43 (CH₂), 39.48 (C), 41.87 (CH₂), 42.58 (CH₂), 55.62 (CH), 58.68 (CH), 83.19 (C), 87.94 (C), 113.06 (CH₂), 141.92 (CH), 169.82 (CO), 170.23 (CO).
ESI-MS (MeOH): 273 [M - OAc - AcOH], 508, 548 [2M - 40Ac].
GC/MS: 272 [M⁺- OAc - AcOH] (20), 257 [M⁺- OAc - AcOH - CH₃] (50), 244 [M⁺-OAc - AcOH - 2CH₂] (5), 229 [M⁺- OAc - AcOH - 2CH₂ - CH₃] (9), 217 (12), 201 (14), 189 (19), 177 (26), 161 (23),147 (20), 137 (100), 121 (45).

### Preparation of (2):

A suspension of 50 mg (0.282 mmol) PdCl₂ and 1 ml acetonitrile was stirred under an Argon-atmosphere for 5 d at room temperature. Then the solution was added to an asolution of 11.765 g (30 mmol) Sclareoldiacetate (1) in 300 ml abs. THF. The mixture was stirred for 6 h at room temperature and then concentrated in a vacuum. The obtained raw product was solved in 40 ml pentane and purified with a column equiped with silica gel (diameter 3 cm, hight 1 cm, solvent: diethylether, 800 ml). After concentrating under reduced pressure the product (2) was obtained as pail yellow oil; Yield: 11.725 g (100%).
R_{f}= 0.49 (pentane/EtOAc 5:1)
¹H-NMR: 0.71 (s, 3H, CH₃), 0.76 (s, 3H, CH₃), 0.80 (s, 3H, CH₃), 0.92 (m, 2H), 1.00-1.72 (m, 7H), 1.39 (s, 3H, CH₃), 1.65 (s, 3H, CH₃), 1.91-2.10 (m, 4H), 1.87 (s, 3H, CH₃), 1.99 (s, 3H, CH₃), 2.50-2.60 (m, 1H), 4.51 (d, J = 7.1 Hz, 1H), 5.26 (dd, J = 6.4, 7.0 Hz, 1H).

### Preparation of (3):

In a 500 ml threenecked flask a solution of 12.800 g (32.6 mmol) (2) in 250 ml abs. CH₂Cl₂ was added. This solution has been cooled to -70°C, and at this temperature, ozone gas from a O₃-generator has been introduced over 1 h. The reaction mixture changed to a green/blue colour. Then, the flask was flashed with nitrogen gas till disappearance of the blue color. Then 12.8 g dimethylsulfid was added and the mixture was stirred overnight. After concentration under reduced pressure a yellow product has been isolated in amounts of 16.604 g. It was purified by means of column chromatography (column with silica gel; diameter 5 cm, hight 50 cm; solvent: pentane/acetoacetate); Yield: 6.890 g (66%).
R_{f} = 0.30 (pentane/EtOAc 5:1)
¹H-NMR: 0.76 (s, 3H, CH₃), 0.82 (s, 3H, CH₃), 0.85 (s, 3H, CH₃), 0.86-1.00 (m, 2H), 1.08-1.43 (m, 4H), 1.45 (s, 3H, CH₃), 1.48-1.77 (m, 7H), 1.91 (s, 3H, CH₃), 2.12 (s, 3H, CH₃), 2.45-2.66 (m, 3H).
¹³C-NMR: 15.51 (CH₃), 18.21 (CH₂), 19.59 (CH₂), 19.91 (CH₂), 20.39 (CH₃), 21.38 (CH₃), 22.95 (CH₃), 29.85 (CH₃), 33.10 (C), 33.29 (CH₃), 38.75 (CH₂), 39.46 (C), 39.61 (CH₂), 41.80 (CH₂), 46.64 (CH₂), 55.60 (CH), 58.04 (CH), 88.03 (C), 170.04 (CO), 209.21 (CO).

### Preparation of (4):

6.890 g (21.36 mmol) of compound (3) was dissolved in 40 ml DMSO and, in a 100 ml flask combined with 2.690 g (32 mmol) NaHCO₃ . This misture has been stirred under an Argon-atmosphere at 150°C for 6 h. After the mixture has been cooled to room temperature 200 ml water was added, and the resulting suspension was extracted with Et₂O (2 x 200 ml). The organic phase was separated, washed with 200 ml water and 50 ml saturated NaCl-solution, dried with Mg₂SO₄ and concentrated under reduced pressure. The raw material (5.428 g, yellow oil) was purified by means of column chromatography (solvent: pentane/EtOAc 10:1). (4) was obtained in amounts of 5.233 g (93%).
R_{f}= 0.57 (pentane/EtOAc 5:1)
¹H-NMR: 0.62 (s, 3H, CH₃), 0.73 (s, 3H, CH₃), 0.79 (s, 3H, CH₃), 0.76-1.55 (m, 10H), 1.61-1.92 (m, 3H), 2.03 (s, 3H, CH₃), 2.18-2.35 (m, 2H), 2.45-2.56 (m, 1H), 4.36 (s, 1H), 4.75 (d, J = 1.0 Hz, 1H).
¹³C-NMR: 14.29 (CH₃), 17.51 (CH₂), 19.34 (CH₂), 21.69 (CH₃), 24.43 (CH₂), 30.02 (CH₃), 33.56 (C), 33.61 (CH₃), 38.26 (CH₂), 38.96 (CH₂), 39.76 (C), 42.11 (CH₂), 42.91 (CH₂), 55.48 (CH), 56.26 (CH), 106.29 (CH₂), 148.35 (C), 209.51 (CO).
GC/MS: 262 [M⁺] (20), 247 [M⁺- CH₃] (17), 244 (26), 229 (39), 204 [M⁺- CH₃CO - CH₃] (46), 191 [M⁺- CH₃COC₂H₄] (26), 189 [M⁺- CH₃CO-2CH₃] (26), 177 [M⁺ - CH₃COC₂H₄-CH₂] (38), 159 (27), 147 [M⁺- CH₃COC₂H₄ - 2CH₃-CH₂] (20), 137 (100), 121 (26), 107 (60), 95 (80).

### Preparation of (1) from (4):

5.1 g (19.43 mmol) (4) was dissolved in 120 ml dichlormethane and in a 500 ml twonecked flask cooled to -78°C. Then, O₃ was introduced under stirring. After introduction of O₃ the mixture has been stirred further at -78°C for approximately 45 min. The flask was then flushed with Nitrogen gas and 6.15 g triphenylphosphine has been added and the misture was stirred 2 h at -78 °C. After bringing the temperature to room temperature the mixture was concentrated under reduced pressure and a yellow oil was obtained in amounts of 12.7 g. The raw material was purified by means of column chromatography (solvent: pentane/EtOAc 10:1). Yield: 3.230 g (63%). Appearance: colorless oil.
R_{f}= 0.19 (pentane/EtOAc 5:1)
¹H-NMR: 0.75 (s, 3H, CH₃), 0.87 (s, 3H, CH₃), 0.98 (s, 3H, CH₃), 1.17-1.33 (m, 2H), 1.42-1.70 (m, 6H), 1.76-1.88 (m, 2H), 2.03-2.14 (m, 2H), 2.12 (s, 3H, CH₃), 2.15-2.35 (m, 2H), 2.39-2.48 (m, 1H), 2.57-2.69 (m, 1H).
¹³C-NMR: 14.55 (CH₃), 16.11 (CH₂), 18.96 (CH₂), 21.66 (CH₃), 23.95 (CH₂), 29.89 (CH₃), 33.50 (C), 33.67 (CH₃), 39.10 (CH₂), 41.84 (CH₂), 42.57 (CH₂), 42.68 (C), 42.76 (CH₂), 54.12 (CH), 63.14 (CH), 209.22 (CO), 212.34 (CO).
GC/MS: 264 [M⁺] (10), 249 [M⁺ - CH₃] (100), 231 (15), 179 (13), 173 (11), 137 (16), 121 (20),109 (15), 95 (23).

## Claims

1. Method for the preparation of (+)-Totarol, **characterized in that** a compound
according to formula (I) 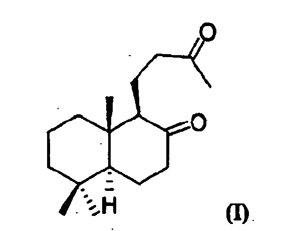 is reacted in an aldol addition and subsequent dehydration to form (II); 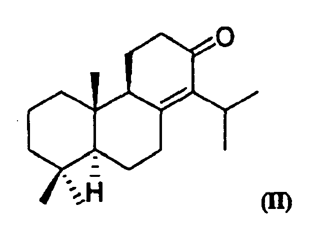 and, as second step the compound (II) is suspended in acetonitrile and then reacted under an oxygen free atmosphere with a blend of LiBr and CuBr₂, heated to reflux to form (+)-Totarol (III)

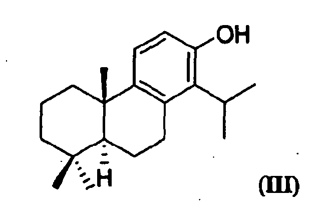

2. Method according to claim 1, **characterized in that** compound (I) is derived from labdan-type diterpens, especially those selected from the group (-)-Sclareol, Manool or epi-Manool, preferably from (-)-Sclareol.

3. Method according to claim 2, **characterized in that**
(i) (-)-Sclareol is acetylated to form diacetylsclareol (1) 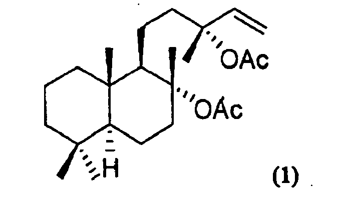
(ii) by a palladium catalyzed rearrangement (1) is converted into isodiacetylsclareol (2) 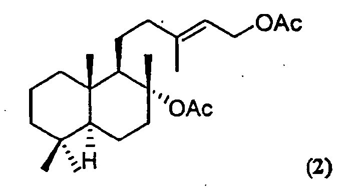
(iii) the iso-diacetylsclareol (2) is subjected to cleavage by ozonolysis to form a ketoacetate derivative (3) 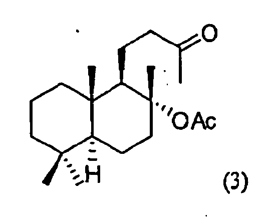
(iv) the ketoacetate derivate (3) is reacted with NaHCO₃ to form the keto-olefine (4), and 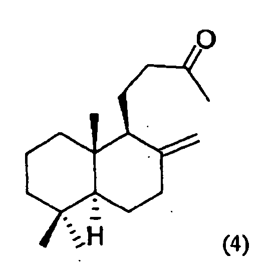
(v) the keto-olefine (4) is converted into (I) by ozonolysis.

## Patentansprüche

1. Verfahren zur Herstellung von (+)-Totarol, **dadurch gekennzeichnet, dass** eine
Verbindung gemäß Formel (I) 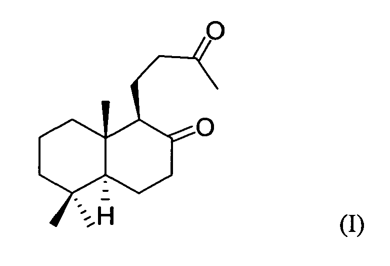 im Laufe einer Aldol Addition und anschließender Dehydratation zu einer Verbindung gemäß Formel (II) wird 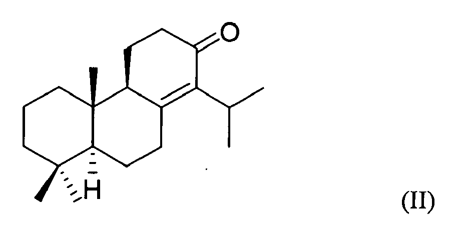 und, als zweiter Schritt die Verbindung II in Acetonitril gelöst wird und dann in einer sauerstoffreien Atmosphäre mit einer Mischung aus Lithiumbromid und Kupferbromid unter Rückfluss erwärmt wird, um (+)- Totarol (III) zu bilden.

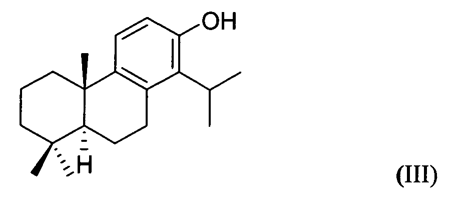

2. Verfahren gemäß Anspruch 1, **dadurch gekennzeichnet, dass** die Verbindung (I) abstammt von Diterpenen des Labdantyps, vorzugsweise solche ausgewählt aus der Gruppe (-)-Sclareol, Manool, oder epi-Manool, vorzugsweise von (-)-Sclareol.

3. Verfahren gemäß Anspruch 2 **dadurch gekennzeichnet, dass**
i) (-)-Sclareol acetyliert wird zu Diacetylsclareol (1) 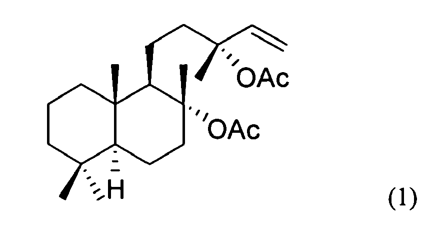
ii) welches durch eine Paladium katalysierte Umlagerung von (1) in Isodiacetylsclareol (2) überführt wird 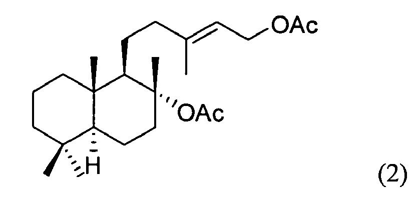
iii) das Isodiacetylsclareol (2) wird Gegenstand einer Spaltung durch Ozonolyse, um das Ketoacetat-Derivat (3) zu bilden 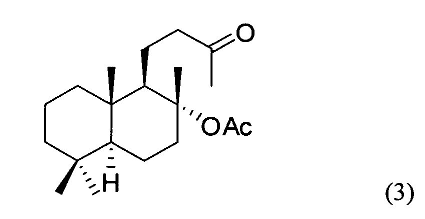
iv) das Ketoacetat-Derivat (3) wird mit Natriumhydrogencarbonat umgesetzt, um das Ketoolefin (4) zu bilden und 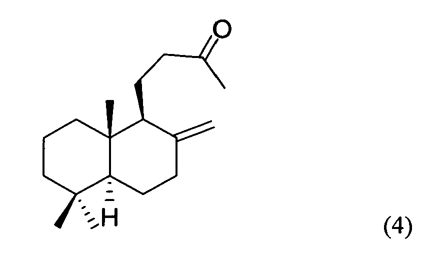
v) das Ketoolefin (4) wird umgewandelt in (I) durch Ozonolyse.

## Revendications

1. Procédé de préparation de (+)-Totarol, **caractérisé en ce qu'**un composé répondant à la formule (I) 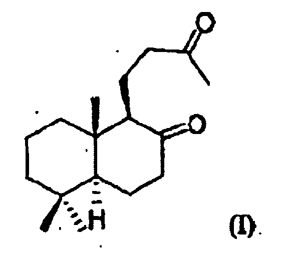 est mis à réagir dans une addition aldol et ensuite soumis à une déshydratation pour former (II) : 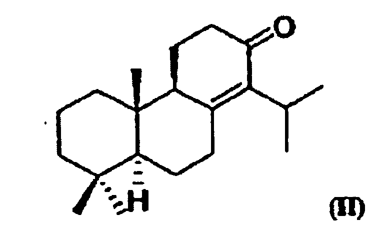 et, dans une deuxième étape, le composé (II) est mis en suspension dans de l'acétonitrile puis mis à réagir sous une atmosphère sans oxygène avec un mélange de LiBr et de CuBr₂, chauffé au reflux pour former le (+)-Totarol (III)

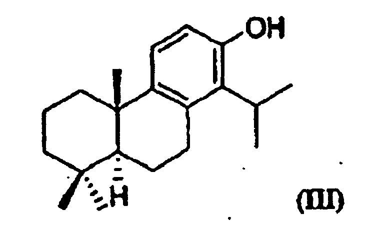

2. Procédé selon la revendication 1, **caractérisé en ce que** le composé (I) est dérivé de diterpènes de type labdane, notamment ceux choisis dans le groupe constitué par le (-)-Sclaréol, le Manool ou l'épi-Manool, de préférence le (-)-Sclaréol.

3. Procédé selon la revendication 2, **caractérisé en ce que**
(i) le (-)-Sclaréol est acétylé pour former le di-acétylsclaréol (1) 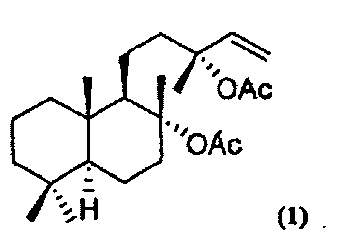
(ii) par une transposition catalysée au palladium (1) est converti en isodiacétylsclaréol (2) 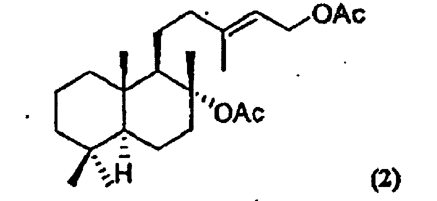
(iii) l'iso-diacétylsclaréol (2) est soumis à un clivage par ozonolyse pour former un dérivé cétoacétate (3) 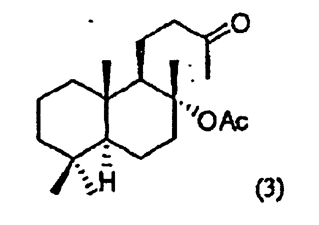
(iv) le dérivé cétoacétate (3) est mis à réagir avec NaHCO₃ pour former la céto-oléfine (4), et 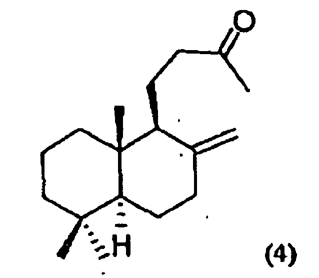
(v) la céto-oléfine (4) est convertie en (I) par ozonolyse.
